Europäisches Patentamt

European Patent Office

Office européen des brevets

(11) Publication number: 0 226 979
A2

# EUROPEAN PATENT APPLICATION

(21) Application number: 86117332.6

(22) Date of filing: 12.12.86

(51) Int. Cl.⁴: **C12N 15/00** , C12P 21/00

(30) Priority: 14.12.85 JP 280259/85

(43) Date of publication of application:
01.07.87 Bulletin 87/27

(84) Designated Contracting States:
CH DE FR GB LI

(71) Applicant: **Chisso Corporation**
**6-32, Nakanoshima 3-chome Kita-ku**
**Osaka-shi Osaka-fu(JP)**

(72) Inventor: **Sakaki, Yoshiyuki**
**12-21-402, Takamiya 1-chome**
**Minamiku Fukuokashi Fukuokaken(JP)**
Inventor: **Inouye, Satoshi**
**10-3, Otsutomocho Kanazawaku**
**Yokohamashi Kanagawaken(JP)**

(74) Representative: **Kraus, Walter, Dr. et al**
**Patentanwälte Kraus, Weisert & Partner**
**Thomas-Wimmer-Ring 15**
**D-8000 München 22(DE)**

(54) Process for producing the protein of an aequorin activity by using a photoprotein aequorin gene.

(57) An efficient biosynthetic process of a natural type or confluent type photoprotein aequorin is provided, which process comprises using a bacterial body transformed by inserting a substantially isolated gene pAQ 440 specifying the biosynthesis of jelly fish photoprotein aequorin into a promoter-containing plasmid.

EP 0 226 979 A2

## Process for producing a protein of an aequorin activity by using a photoprotein aequorin gene

BACKGROUND OF THE INVENTION

1. Field of the Invention

This invention relates to a process for producing a protein of an aqueorin activity by using a photoprotein aequorin gene. More particularly, it relates to a process for producing the above-mentioned protein by using a bacterial body obtained by substantially isolating the above-mentioned gene, inserting the resulting gene into a promoter-containing plasmid and thereby transforming the plasmid.

Description of the Related Art

Aequorin is a photoprotein of a photogenic jelly fish growing in a northern district of the west coast in America, and when one molecule of aequorin is specifically bonded two or three mols of $Ca^{2+}$, aequorin oxidizes coelenterazine as an emitter to cause luminescence. On the other hand, in view of the fact that $Ca^{2+}$ is indispensable for the luminescence of aequorin, it is possible to specifically determine the quantity of $Ca^{2+}$ by the use of aequorin; thus it is possible to measure the concentration of $Ca^{2+}$ in a trace of about $10^{-7}$ M.

At present, there are existent many $Ca^{2+}$-dependent enzymes in the living body and there is a possibility of causing a serious disease due to $Ca^{2+}$ deficiency; thus the aequorin has a possibility of being broadly used for clinical inspection, and further, by injecting it into cells, it is possible to apply it to measurement of $Ca^{2+}$ concentration inside the cells, cold-heat light source, etc.

However, the growing place of the jelly fish is limited to a northern district of the west coast in America; the season of its generation is restricted only to summer season; and the yield is only about one mg per 10,000 jelly fishes so that its production quantity is insufficient and a stabilized supply cannot be ensured.

The present inventors previously made extensive research in order to solve the above-mentioned problems, and as a result, by employing a technique of gene engineering, isolated and prepared the aequorin gene substantially specifying the formation of aequorin protein, as a cDNA clone from mRNA according to Okayama-Berg method.

Namely, by employing a recombinant DNA procedure, we prepared cDNA clone of jelly fish photoprotein aequorin, and named this cDNA clone a plasmid pAQ 440 (see Japanese patent application No. Sho 59-176125/1984; the invention of the application will hereinafter be referred to as "the prior invention").

Further, the previous invention is directed to a process for producing a photoprotein aequorin characterized by cultivating a host (e.g. Escherichia coli) modified so as to contain the deoxyribonucleotide arrangement of the photoprotein aequorin or a functional equivalent to the deoxyribonucleotide arrangement.

However, since the above-mentioned cDNA clone plasmid pAQ 440 has no particular promoter, it has been difficult to efficiently produce the photoprotein aequorin by merely cultivating the pAQ 440 as described above.

Thus, the present inventors have further continued research in order to solve the above-mentioned technical problem relative to the previous invention, and as a result, have found that when a plasmid-containing bacterial body obtained by inserting the aequorin gene into a promoter-containing "expression vector" is used, it is possible to efficiently produce a natural type or confluent type photoprotein aequorin.

SUMMARY OF THE INVENTION

As apparent from the foregoing, an object of the present invention is to provide an efficient biosynthetic process of a natural type or confluent type photoprotein aequorin. Another object of the present invention is to provide a process for producing a promoter-containing bacterial body, which makes the above biosynthesis possible.

The present invention has the following constitutions and effectiveness:

(1) A process for producing a protein of an aequorin activity which process comprises using a bacterial body transformed by inserting a substantially isolated gene pAQ 440 specifying the biosynthesis of jelly fish photoprotein aequorin into a promoter-containing plasmid.

(2) A process for producing a protein of an aequorin activity according to the item (1) wherein lac , tac or trp originated from Escherichia coli is used as said promoter to obtain a confluent type or natural type aequorin protein.

(3) A process for producing a protein of an aequorin activity according to the item (1) wherein PL promoter of λ phage is used as said promoter to obtain a confluent type or natural type aequorin protein.


BRIEF DESCRIPTION OF THE DRAWING

Figs. 1 -3 each show a view illustrating the process of the present invention.

Fig. 1 shows a flowsheet illustrating construction of tac-expression vector and a process of inserting aequorin cDNA into the expression vector.

Fig. 2 shows a chart illustrating the structural view of the promoter region of vector piQ 5 for a natural type aequorin synthesis.

Fig. 3 shows a chart illustrating the structure of inserted clone of aequorin cDNA gene into lac promoter-expression vector.


DETAILED DESCRIPTION OF PREFERRED EMBODIMENTS

The above constitutions and effectiveness of the present invention will be described below in detail.

① Insertion of aequorin gene into an expression vector vector having a promoter:

In the present invention, as the expression vector into which aequorin gene is inserted, the one wherein the promoter has been subjected to cloning is used.

An example of the vector is pUC 9.

As the promoter, for example, PL of lac, tac, trp or λ phage originated from Escherichia coli may be used.

The promoter is prepared as follows:

pDR 540 having a promoter is digested with Bam HI -Hind III (a restriction enzyme), and the resulting fragment containing the promoter after the digestion is subjected to separation-extraction for example by electrophoresis in 8% acrylamide gel.

Using the thus extracted promoter, cloning is carried out at the site of Bam HI-Hind III to obtain an expression vector (piC9).

The Hind III site portion of the expression vector (piC 9) is then removed as follows:

The vector is digested at the Hind III, followed by repairing the ends with E. Coli DNA polymerase - (Klenow fragment) in the presence of dATP, dGTP, dCTP and dTTP and bonding both the ends by means of T₄ ligase to obtain an expression vector (piC 10).

Next, as preparation for linking the promoter in (piC 10) to aequorin cDNA gene, cloning is carried out into (piC 10) with synthetic nucleotides as linkers.

Namely, synthetic nucleotides AR (5′ GATCGATGGTCA 3′) and AQ (5′ AGCTTGACCATC 3′) are synthesized according to a known method, respectively, followed by annealing these, thereafter phosphorylating the 5′ end thereof with T4-nucleotide kinase in the presence of ATP, then cloning the resulting phosphorylated synthetic nucleotides in the form in which the linkers are repeated at the Bam H-Eco RI site of the previously prepared piC 10, to Prepare piC 11.

Next, Hin d III-Eco RI fragment is separated and purified from aequorin cDNA clone pAQ 440 (with regard to this, see the above-mentioned Japanese patent application No. Sho 59-176125.1984), followed by inserting the resulting fragment into the site of Hind III-Eco RI of piC 11, to obtain a bacterial body containing piQ 5 i.e. a promoter having transformed the gene pAQ 440.

This piQ 5 has a capability of producing a natural type aequorin protein.

② Insertion of aequorin gene into a lac promoter-containing expression vector (pUC9, 9-1, 9-2) for producing a confluent type aequorin protein:

In the same manner as that described in the above paragraph ① , Pst I-Eco RI and Hind III-Eco RI fragments are separated and purified from cDNA clone pAQ 440.

Further, expression vectors pUC 9, 9-1, 9-2 having a promoter of lac prepared in the same method as that described in the above paragraph ① is obtained.

3

Further, clonings of the above respective fragments are carried out at the restriction enzyme sites of the above respective vectors, whereby piQ 9PE, 9-IPE, 9-2PE, 9-HE and 9-2HE are prepared.

These are all ruled under lac promoter, and are confluent type aequorin proteins having 8-amino acid residual group at N-end.

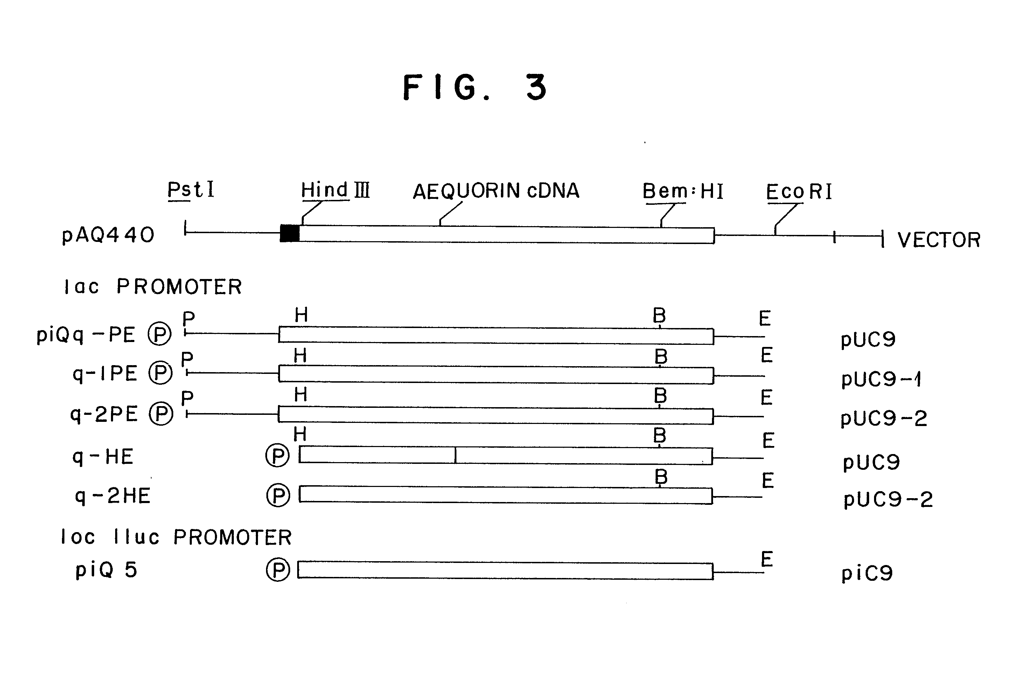

The respective structures are shown in Fig. 3. ③ Production of a protein having aequorin activity by means of Escherichia coli:

The respective plasmids subjected to cloning into the expression vectors obtained in the above paragraphs ① and ② are transformed into e.g. E. coli (D 1210) strain, whereby it is possible to make the resulting strain produce aequorin protein.

Namely, for example, to a suitable quantity (10 mℓ) of LB broth containing a suitable quantity (50 μg/mℓ) of Ampicillin is added "a bacterial body retaining a plasmid" for example, in a quantity of 1/100 of that of the broth cultivated for 12 hours, followed by cultivating the mixture at 37°C for 2 hours, successively adding an inducing reagent IPTG (isopropyl-$\beta$-thiogalactopyranoside) so as to give a final concentration of 1 mM and successively cultivating the mixture at 37°C for 2 hours.

The thus obtained culture is treated at 5,000 rpm for 10 minutes by means of a centrifuge (Hitachi RP 20, tradename of product manufactured by Hitachi Seisakusho Company) to collect the resulting bacterial bodies, followed by washing the resulting bacterial bodies with M9 salt solution (5 ℓ).

The thus washed bacterial bodies are treated as follows to obtain an enzyme solution to be measured

Namely, the collected bacterial bodies are first dissolved in a 20 mM Tris-HCℓ buffer (pH: 7.6) (2.5 mℓ containing EDTA (10 mM), followed by subjecting the resulting solution to ultrasonic treatment for 60 seconds to break the bacterial bodies, and successively subjecting the resulting material to centrifugal treatment at 10,000 rpm for 10 minutes, to use the resulting supernatant as the enzyme solution to be measured.

The method of measuring the aequorin activity is carried out for example as follows:

A substrate coelenterazine (6 μg) and 2-mercaptoethanol (10 μℓ) are added to the above enzyme solution (1 mℓ), followed by allowing the mixture to stand on ice for 2 -3 hours, transferring it into a reaction cell in a photocell measurement apparatus, injecting 20 mM CaCℓ₂ (1.5 mℓ) and measuring the resulting luminescence.

A measurement example is shown in Table 1 described in Example 3.

As apparent from the Table, the above-mentioned respective vectors have a very low luminescent ability, irrespective of whether or not the inducting reagent IPTG is added to the fluid to be measured, whereas the cultures obtained by subjecting the aequorin gene relative to the present invention to cloning downstream from the promoter, have a luminescent ability except for piQ 9-HE, and the ability increases with a leap by adding IPTG.

According to the process of the present invention, since the gene of the photoprotein aequorin is used by inserting it into a plasmid having a promoter contained therein, followed by subjecting the resulting material to transformation into bacterial bodies, it is possible to efficiently produce a large quantity of confluent type or natural type photoprotein aequorin.

The present invention will be described in more detail by way of Examples.

Example 1

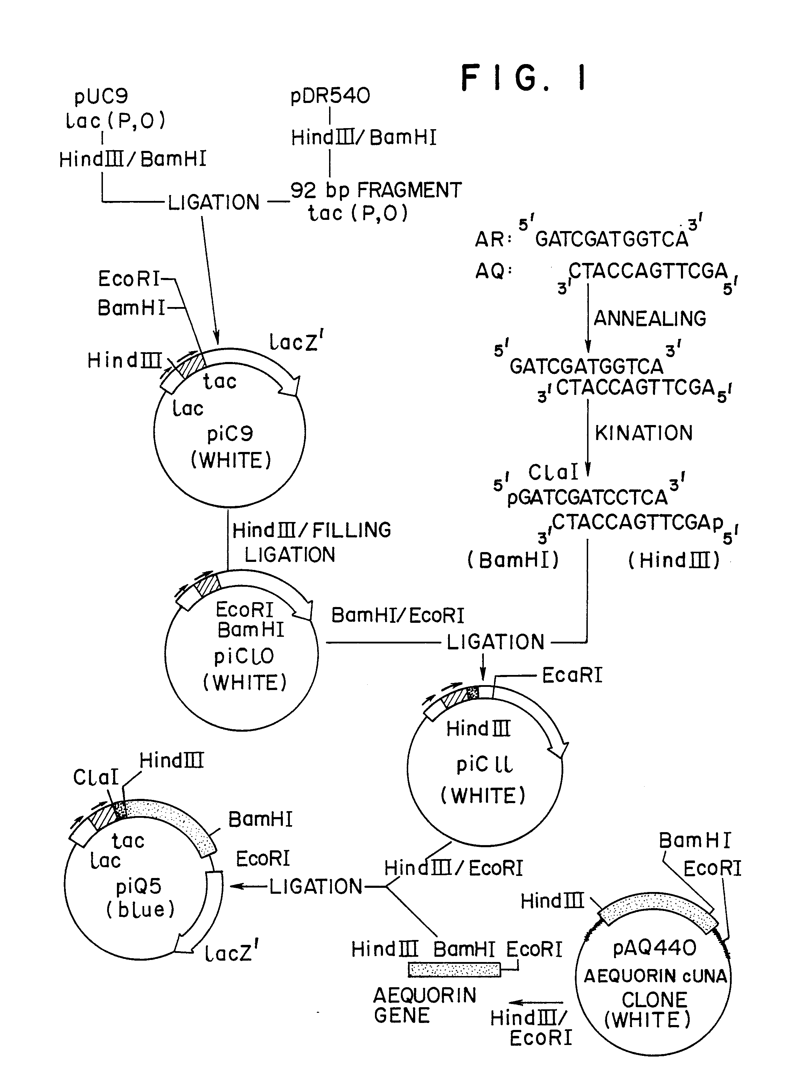

Insertion of aequorin gene into an expression vector (piC 9) having a tac promoter (see Fig. 1)

(a) Preparation of piC 10:

pDR 540 (made by PL pharmacie Company) having a tac promoter was digested with a restriction enzyme Bam HI-Hind (III), followed by separating a fragment containing a tac promoter of 92 bp by electrophoresis in 8% acrylamide, thereafter carrying our cloning at Bam HI-Hind III site of a vector pUC 9 to prepare piC 9, thereafter, in order to remove the Hind III site portion of the expression vector piC 9, first carrying out Hin d III digestion, successively repairing the ends with E. coli DNA polymerase (Klenow fragment) in the presence of dATP, dGTP, dCTP and dTTP and thereafter bonding both the ends by T₄ ligase to prepare piC 10.

(b) Preparation of piC 11:

On the other hand, in order to link the aequorin cDNA gene to the promoter, synethic nucleotide AR (5′ GATCGATGGTCA 3′)and AQ (5′ AGCTTGACCATC 3′) as linkers were prepared, followed by annealing the resulting material, thereafter phosphorylating 5′ ends with T₄ nucleotide kinase in the presence of ATP, and carrying out cloning in the form in which the linkers were repeated at the Bam HI-Eco RI site of piC 10 previously prepared, to prepare piC 11.

(c) Preparation of piQ 5:

The Hind III-Eco RI fragment was separated and purified from the aequorin cDNA clone pAQ 440, followed by inserting the fragment into the Hind III-Eco RI site to prepare piQ 5.

The piQ 5 prepared according to the above process can produce a natural type aequorin protein.

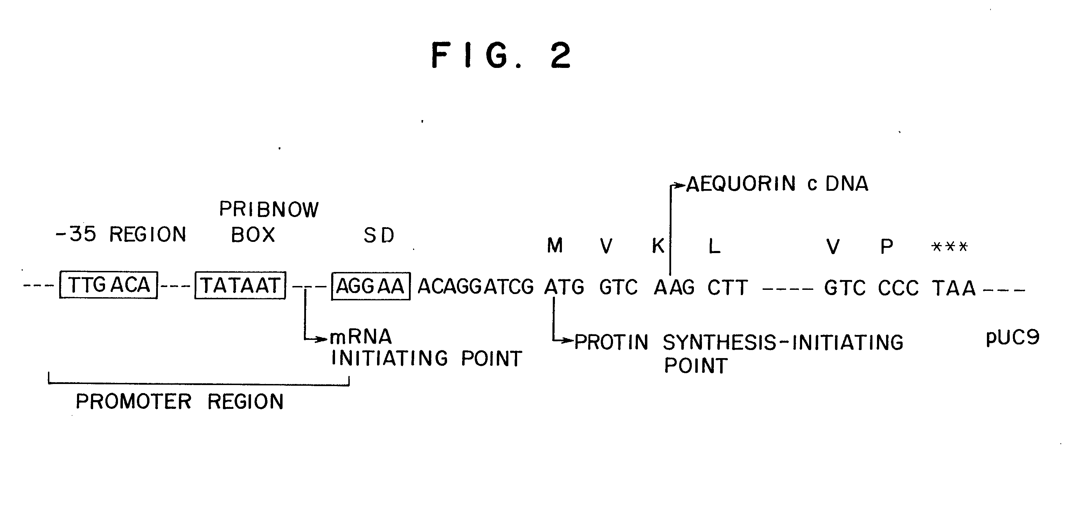

Fig. 2 shows the structure in the vicinity of the tac promoter and the aequorin gene. In this figure, -35 region refers to the recognition site of RNA polymerase and Pribnow box refers to the binding site of RNA polymerase. Further, SD refers to the binding site of ribosome.

## Example 2

Insertion of a confluent type aequorin into a developed vector having a lac promoter (pUC 9, 9-1, 9-2):

A Pst I-Eco RI, Hind III-Eco RI fragment was separated and purified from CDNA clone pAQ 440, followed by carrying out cloning at the respective restriction enzyme site of pUC 9, 9-1, 9-2, having a lac promoter to prepare piQ 9PE, 9-IPE, 9-2PE, 9-HE and 9-2HE. These are all ruled under lac promoter and are confluent type aequorin proteins having 8-amino acid residual group at N end.

The structures of the above respective products are shown in Fig. 3. In this figure, Ⓟ refers to promoter; B refers to Bam HI; and E refers to Eco RI.

## Example 3

Production process by way of Escherichia coli:

The plasmid prepared by carrying out cloning into the expression vector in the above paragraph (1) and (2) was subjected to transformation into E-coli (D 1210) strain to produce aequorin protein.

Namely, to a LB broth (10 mℓ) containing Ampicillin in a concentration of 50 μg/mℓ) was added the above-mentioned bacterial body after cultivation for 12 hours (note: plasmid-retaining bacterial body) in a quantity of 1/100 of that of the broth, followed by cultivating the mixture at 37°C for 2 hours, adding to the resulting culture, an inducing reagent IPTG (isopropyl-β-thiogalactopyranoside) so as to give a final concentration of 1mM, and further cultivating the resulting mixture at 37°C for 2 hours.

From the resulting culture were collected bacterial bodies at 5,000 rpm for 10 minutes by means of a centrifuge (Hitachi RP 20 (tradename of product manufactured by Hitachi Seisakusho Company )), followed by washing the bacterial bodies with a M9 salt (5 mℓ), dissolving the resulting washed material in 20 mM Tris HCℓ buffer (pH: 7.6) (2.5 mℓ) containing 10 mM EDTA, thereafter breaking the bacterial bodies by ultrasonic treatment (60 seconds), subjecting the resulting material to centrifugal preparation treatment at 10,000 rpm for 10 minutes and using the resulting supernatant as an enzyme solution to be measured.

As to the measurement method, a substrate coelenterazine (6 μg) and 2-mercaptoethanol (10 μℓ) were added to the enzyme solution (1 mℓ), followed by allowing the mixture to stand on ice for 2 -3 hours, thereafter transferring it into a reaction cell in a photocell measuring apparatus, and further injecting 20 mM CaCℓ₂ (1.5 mℓ) to measure the resulting luminescence.

The measurement results are shown in Table 1.

According to the Table, about 10 ng of aequorin protein in 1 mℓ of the culture fluid of this Example was produced as calculated from natural aequorin protein by way of induction of an inducting reagent IPTG.

Table 1

Production process

of aequorin protein in Escherichia coli

| Plasmid | IPTG ( 1 mM ) | |
|---|---|---|
| | − | + |
| Control(-plasmid) | 0 | 0 |
| pUC9 | 0 | 0 |
| pUC9 − 1 | 0 | 0 |
| pUC9 − 2 | 0 | 0 |
| piC11 | 0 | 0 |
| pAQ440 | 0.7 | 0.6 |
| piQ9 − PE | 1.9 | 176.5 |
| piQ9 − 1PE | 0.4 | 38.9 |
| piQ9 − 2PE | 0.5 | 51.2 |
| piQ9 − HE | 0 | 0 |
| piQ9 − 2HE | 0.7 | 250.8 |
| piQ5 | 10.5 | 325.8 |

$\times 10^8$ quanta /second

**Claims**

1. A process for producing a protein of an aequorin activity which process comprises using a bacterial body transformed by inserting a substantially isolated gene pAQ 440 specifying the biosynthesis of jelly fish photoprotein aequorin into a promoter-containing plasmid.

2. A process for producing a protein of an aequorin activity according to Claim 1 wherein lac, tac or trp originated from Escherichia coli is used as said promoter to obtain a confluent type of natural type aequorin protein.

3. A process for producing a protein of an aequorin activity according to Claim 1 wherein PL promoter of λ phage is used as said promoter to obtain a confluent type or natural type aequorin protein.

# FIG. I

pUC9
Lac(P,O)
HindⅢ/BamHI

pDR540
HindⅢ/BamHI

— LIGATION — 92 bp FRAGMENT
tac(P,O)

EcoRI
BamHI
HindⅢ

tac
Lac
piC9
(WHITE)

LacZ'

HindⅢ/FILLING
LIGATION

EcoRI
BamHI
piCLO
(WHITE)

BamHI/EcoRI
— LIGATION —

AR: 5' GATCGATGGTCA 3'
AQ:    3' CTACCAGTTCGA 5'

ANNEALING

5' GATCGATGGTCA 3'
   3' CTACCAGTTCGA 5'

KINATION

ClaI
5' pGATCGATCCTCA 3'
      3' CTACCAGTTCGAp 5'
(BamHI)      (HindⅢ)

EcaRI

HindⅢ
piCLL
(WHITE)

HindⅢ/EcoRI

ClaI  HindⅢ

tac
Lac
piQ5
(blue)

BamHI
EcoRI

— LIGATION —

LacZ'

HindⅢ  BamHI  EcoRI

AEQUORIN
GENE

HindⅢ/
EcoRI

BamHI
EcoRI

HindⅢ

pAQ440
AEQUORIN cUNA
CLONE
(WHITE)

# FIG. 2

PRIBNOW                                                    ►AEQUORIN c DNA

-35 REGION    BOX            S D                M    V    K   L      V   P   ***

--- [TTGACA] --- [TATAAT] --,-- [AGGAA] ACAGGATCG ATG GTC AAG CTT ---- GTC CCC TAA ---

                            └►mRNA                └►PROTIN SYNTHESIS-INITIATING   pUC9
                              INITIATING POINT       POINT

PROMOTER REGION

# F I G. 3

0 226 979